# EUROPEAN PATENT APPLICATION

(11) **EP 4 555 934 A1**
(43) Date of publication of application: **21.05.2025**
(21) Application number: 24214002.8
(22) Date of filing: 19.11.2024
(51) Int. Cl.: A61B 6/00

(54) **RADIOGRAPHY SYSTEM**

(30) Priority: 20.11.2023 JP 2023196966
(71) Applicant: FUJI-FILM Corporation, Minato-ku Tokyo 106-8620 (JP)
(72) Inventor: HORIUCHI, Hisatsugu, Kaisei-machi (JP); KOBAYASHI, Takeyasu, Kaisei-machi (JP); MORI, Kyohei, Kaisei-machi (JP); MATSUURA, Masayoshi, Kaisei-machi (JP); NISHINO, Naoyuki, Kaisei-machi (JP)
(74) Representative: Dehns Germany Partnerschaft mbB

(57) **Abstract**

A radiography system includes a radiation source unit (40) and a panel unit (60). The radiation source unit includes a radiation emitting unit (42) and a radiation source base part (50). The panel unit includes a panel (62) that detects radiation, and a panel base part (70). A floor surface is defined as a projection surface, and a projection region of the radiation source base part and a projection region of the panel base part have a common shape. The radiation source unit and the panel unit can be disposed in a close state where the floor surface is defined as a projection surface and at least one of a projection region of the radiation source unit and the projection region of the panel base part or a projection region of the panel unit and the projection region of the radiation source base part at least partially overlap with each other.

## Description

### BACKGROUND OF THE INVENTION

### 1. Field of the Invention

The present disclosure relates to a radiography system.

### 2. Description of the Related Art

In the related art, a radiography system in which the position of each a radiation emitting unit including a radiation source and a panel detecting radiation emitted from the radiation emitting unit are freely movable is known as a radiography system that captures a radiographic image of a subject.

For example, JP2000-166906A discloses that an X-ray source and an X-ray receiver are disposed on a plurality of robot arms capable of being electrically displaced, respectively, and are freely displaced in a space by controlling positions of the robot arms. Further, for example, JP2023-503638A discloses a portable X-ray system that includes two or more wheels or a moving mechanism.

### SUMMARY OF THE INVENTION

Incidentally, in a case where a portable radiography system is operated, it is considered to store a device in an appropriate place when the device is not used and to carry out the device only when the device is used. Therefore, in recent years, there has been a demand for a radiography system of which storage efficiency can be improved.

The present disclosure provides a radiography system of which storage efficiency can be improved.

According to an aspect of the present disclosure, there is provided a radiography system that comprises a radiation source unit and a panel unit. The radiation source unit includes a radiation emitting unit that includes a radiation source, and a radiation source base part that supports the radiation emitting unit and is capable of traveling on a floor surface. The panel unit includes a panel that detects radiation emitted from the radiation emitting unit, and a panel base part that supports the panel and is capable of traveling on the floor surface. In a case where the floor surface is defined as a projection surface, a projection region of the radiation source unit is defined as an entire radiation source region, a projection region of the radiation source base part is defined as a radiation source base region, a projection region of the panel unit is defined as an entire panel region, and a projection region of the panel base part is defined as a panel base region, the radiation source base region and the panel base region have a common shape. The radiation source unit and the panel unit are capable of being disposed in a close state where at least one of the entire radiation source region and the panel base region or the entire panel region and the radiation source base region at least partially overlap with each other.

In the aspect, the entire radiation source region and the panel base region may at least partially overlap with each other in the close state.

In the aspect, in a case where the floor surface is defined as the projection surface and a projection region of the radiation emitting unit is defined as an emitting unit region, the emitting unit region and the panel base region may at least partially overlap with each other in the close state.

In the aspect, the entire panel region and the radiation source base region may at least partially overlap with each other in the close state.

In the aspect, in a case where the floor surface is defined as the projection surface and a projection region of the panel is defined as a panel region, the panel region and the radiation source base region may at least partially overlap with each other in the close state.

In the close state in the aspect, the entire radiation source region and the panel base region may at least partially overlap with each other, and the entire panel region and the radiation source base region may at least partially overlap with each other.

In the aspect, in a case where the floor surface is defined as the projection surface, a projection region of the radiation emitting unit is defined as an emitting unit region, and a projection region of the panel is defined as a panel region, in the close state, the emitting unit region and the panel base region may at least partially overlap with each other and the panel region and the radiation source base region may at least partially overlap with each other.

In the aspect, the radiation source base region and the panel base region may include an overlap region in which the radiation source base region and the panel base region at least partially overlap with each other in the close state.

In the aspect, each of the radiation source base part and the panel base part may include a plurality of wheels disposed in a common arrangement pattern, and, in a case where the floor surface is defined as the projection surface and a projection region of each of the plurality of wheels is defined as a wheel region, some of the wheel regions and the overlap region may at least partially overlap with each other in the close state.

In the aspect, in a case where a direction intersecting a direction in which the radiation source unit and the panel unit face each other on the floor surface in the close state is defined as a width direction, mounting positions of the wheels of the radiation source base part and the wheels of the panel base part may be different from each other in the width direction.

In the close state in the aspect, a part of the wheel regions of the panel base part may enter the radiation source base region and a part of the wheel regions of the radiation source base part may enter the panel base region.

In the aspect, each of the radiation source base part and the panel base part may include one or more front wheels and two or more rear wheels, and at least a part of the front wheels included in any one of the radiation source base part or the panel base part may enter between the rear wheels included in the other of the radiation source base part and the panel base part in the close state.

In the aspect, in a case where a direction intersecting a direction in which the radiation source unit and the panel unit face each other on the floor surface in the close state is defined as a width direction, a width of all the front wheels in the width direction may be smaller than a width of all the rear wheels in the width direction.

In the aspect, the radiation source unit may further include a radiation source support part that extends in a direction intersecting the floor surface and connects the radiation source base part and the radiation emitting unit, and a distance from a connecting portion between the radiation source base part and the radiation source support part to an end portion of the radiation source base part corresponding to the rear wheel may be shorter than a distance from the connecting portion to an end portion of the radiation source base part corresponding to the front wheel in a direction in which the radiation source unit and the panel unit face each other in the close state.

In the aspect, the panel unit may further include a panel support part that extends in a direction intersecting the floor surface and connects the panel base part and the panel, and a distance from a connecting portion between the panel base part and the panel support part to an end portion of the panel base part corresponding to the rear wheel may be shorter than a distance from the connecting portion to an end portion of the panel base part corresponding to the front wheel in a direction in which the radiation source unit and the panel unit face each other in the close state.

In the aspect, the radiation source base region and the panel base region may have a congruent shape.

In the aspect, the radiation source base region and the panel base region may have a rectangular shape.

In the aspect, a total region of the entire radiation source region and the entire panel region may have a rectangular shape in the close state.

In the aspect, at least one of the radiation emitting unit or the panel may be adapted such that a position of at least one of the radiation emitting unit or the panel in a height direction perpendicular to the floor surface is changeable, and the radiation emitting unit and the panel may not interfere with each other in the height direction in the close state.

In the aspect, the radiography system may further comprise at least one processor. The processor may be configured to: acquire difference information indicating a difference between positions of the radiation emitting unit and the panel in the height direction in a separated state where the respective regions do not overlap with each other; and derive a moving distance of at least one of the radiation emitting unit or the panel in the height direction on the basis of the difference information such that a positional relationship between the radiation emitting unit and the panel in the height direction is a predetermined state.

According to the aspect, the storage efficiency of the radiography system of the present disclosure can be improved.

### BRIEF DESCRIPTION OF THE DRAWINGS

Fig. 1 is a diagram showing an example of a schematic configuration of a radiography system.
Fig. 2 is a diagram showing an example of the schematic configuration of the radiography system in supine imaging.
Fig. 3 is a diagram showing an example of the schematic configuration of the radiography system in a close state.
Fig. 4 is a diagram showing an example of projection regions of the radiography system in the close state.
Fig. 5 is a diagram showing the radiography system in the close state that is disposed in a storage place.
Fig. 6 is a diagram showing a radiography system of a comparative example that is disposed in a storage place.
Fig. 7 is a block diagram showing an example of a functional configuration of the radiography system.
Fig. 8 is a block diagram showing an example of an electric configuration of a control device.
Fig. 9 is a diagram showing an example of a schematic configuration of a radiography system according to a first modification example.
Fig. 10 is a diagram showing an example of projection regions of the radiography system according to the first modification example.
Fig. 11 is a diagram showing an example of a schematic configuration of a radiography system according to a second modification example.
Fig. 12 is a diagram showing an example of a configuration of a base part of the second modification example.
Fig. 13 is a diagram showing an example of projection regions of the radiography system according to the second modification example.

### DESCRIPTION OF THE PREFERRED EMBODIMENTS

An embodiment of the present disclosure will be described below with reference to the drawings. In each drawing, the same or equivalent components and parts are denoted by the same reference numerals. Dimensional ratios in the drawings are exaggerated for convenience of description and may be different from actual ratios.

In the following description, for convenience of description, a width direction, a depth direction, and a height direction of a radiography system 10 are indicated by three arrows X, Y, and Z. First, the height direction is indicated by the arrow Z, an arrow Z direction indicated by the arrow Z is defined as an upward direction of the radiography system 10, and a direction opposite thereto is defined as a downward direction. The depth direction is indicated by the arrow Y orthogonal to the arrow Z, a direction indicated by the arrow Y is defined as a rear direction of the radiography system 10, and a direction opposite thereto is defined as a front direction. The width direction is indicated by the arrow X orthogonal to the arrow Z and the arrow Y, a direction indicated by the arrow X is defined as a right direction of the radiography system 10, and a direction opposite thereto is defined as a left direction. Further, in the following description, expressions using "side" such as an upper side, a lower side, a back side, a front side, a right side, and a left side also have the same meanings as expressions using "direction".

Furthermore, in the present embodiment, "perpendicular" refers to perpendicularity in the sense of including an error that is generally allowed in the technical field to which the technique of the present disclosure belongs and does not depart from the spirit of the technique of the present disclosure, in addition to a completely perpendicular direction. In addition, the same applies to "parallel", and "parallel" refers to parallelism in the sense of including an error that is generally allowed in the technical field to which the technique of the present disclosure belongs and does not depart from the spirit of the technique of the present disclosure, in addition to a completely parallel direction.

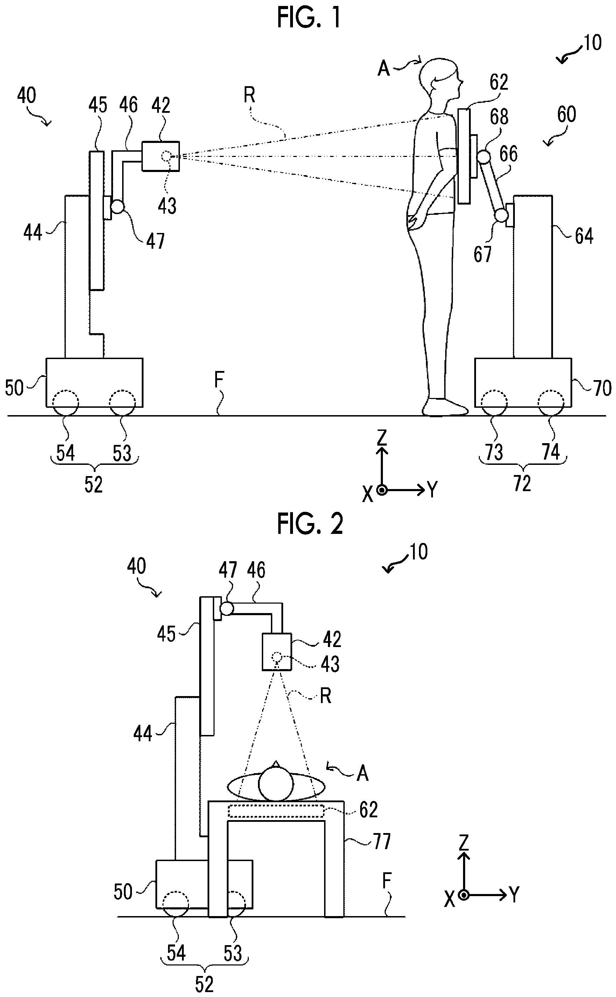

First, an overall configuration of the radiography system 10 according to the present embodiment will be described with reference to Fig. 1. Fig. 1 is a schematic diagram showing an example of the configuration of the radiography system 10. The radiography system 10 is a system that is used to perform radiography, and comprises a portable radiation source unit 40 and a portable panel unit 60. An aspect in which the chest of a subject A in a standing position is imaged is shown in the example shown in Fig. 1.

The radiation source unit 40 comprises a radiation emitting unit 42 that includes a radiation source 43, a radiation source base part 50 that supports the radiation emitting unit 42 and can travel on a floor surface F, and a radiation source support part 44 that connects the radiation source base part 50 and the radiation emitting unit 42. The radiation source unit 40 is a unit that irradiates the subject A with the radiation R (for example, X-rays and gamma rays) generated from the radiation source 43.

The radiation emitting unit 42 irradiates the subject A with the radiation R. The radiation source 43 is accommodated in the radiation emitting unit 42. An irradiation direction of the radiation R generated from the radiation source 43 is defined by an irradiation field limiter (not shown). Irradiation conditions of the radiation R, such as a tube voltage, a tube current, and an irradiation time of the radiation R, to be applied to the radiation source 43 may be, for example, predetermined in an imaging order or may be input by an operator, such as a doctor or a technician.

The radiation source base part 50 includes a radiation source traveling mechanism 52. The radiation source traveling mechanism 52 is a mechanism that allows the radiation source base part 50 to travel and move on the floor surface F. That is, the radiation source unit 40 is adapted such that the position of the radiation source unit 40 on the floor surface F (in an XY direction) can be changed. The radiation source traveling mechanism 52 comprises, for example, front wheels 53 and rear wheels 54 that are provided at a lower portion of the radiation source base part 50 and a motor (not shown) that drives the front wheels 53 and the rear wheels 54. The radiation source traveling mechanism 52 may be adapted to travel by being pushed by, for example, a user, instead of being driven by the motor. The front wheels 53 and the rear wheels 54 are an example of a plurality of wheels of the present disclosure.

The radiation source support part 44 comprises a lifting part 45 that extends in a direction (Z direction) intersecting the floor surface F and can be lifted and lowered in the height direction, and an arm 46 that is connected to the lifting part 45 at a connecting portion 47. The radiation source support part 44 is a part that forms a body part of the radiation source unit 40, and a power supply system for supplying power to the radiation source 43, a controller for controlling the entire radiation source unit 40, a mechanism for driving the arm 46, and the like are accommodated in the radiation source support part 44.

The arm 46 is a part extending from the radiation source support part 44, one end of the arm 46 is mounted on the lifting part 45, and the radiation emitting unit 42 is provided at the other end of the arm 46. The arm 46 is extendable and contractible in the height direction. Further, the connecting portion 47 between the lifting part 45 and the arm 46 is also adapted such that the position of the connecting portion 47 in the height direction can be changed in the lifting part 45. That is, the radiation emitting unit 42 is adapted such that the position of the radiation emitting unit 42 in the height direction (Z direction) perpendicular to the floor surface F can be changed due to the lifting and lowering of the lifting part 45, a change in the position of the connecting portion 47 in the height direction, and the extension and contraction of the arm 46 in the height direction. Furthermore, the arm 46 may be adapted to be extendable and contractible even in the depth direction.

Further, the arm 46 may be adapted to be rotatable about an axis extending in a right-left direction (X direction) at the connecting portion 47 (see Fig. 2). Furthermore, the arm 46 may be adapted to be rotatable about an axis extending in the depth direction (Y direction) at the connecting portion 47.

In the example shown in Fig. 1, the arm 46 is formed in an L shape to include a portion parallel to a side surface of the radiation source support part 44 (extending in an up-down direction) and a portion bent from an upper end of the portion and extending in the depth direction. A form of the arm 46 is not particularly limited. For example, the arm 46 may be a C-shaped arm as viewed from the right side as in Fig. 1, or may be an arm including a plurality of joints.

The panel unit 60 comprises a panel 62 that detects the radiation R emitted from the radiation emitting unit 42, a panel base part 70 that supports the panel 62 and can travel on the floor surface F, and a panel support part 64 that connects the panel base part 70 and the panel 62. The panel unit 60 detects the radiation R with which the subject A is irradiated from the radiation source unit 40 to generate a radiographic image of the subject A.

The panel 62 is a so-called indirect conversion type radiation detector that includes a scintillator for converting the radiation R into visible light and a semiconductor substrate in which pixels generating electric charges in response to visible light are two-dimensionally arranged and detects the radiation R transmitted through the subject A to output a radiographic image. The panel 62 may be a so-called direct conversion type radiation detector including a semiconductor substrate in which pixels generating electric charges in response to the radiation R are two-dimensionally arranged.

The panel base part 70 includes a panel traveling mechanism 72. The panel traveling mechanism 72 is a mechanism that allows the panel base part 70 to travel and move on the floor surface F. That is, the panel unit 60 is adapted such that the position of the panel unit 60 on the floor surface F (in the XY direction) can be changed. The panel traveling mechanism 72 comprises, for example, front wheels 73 and rear wheels 74 that are provided at a lower portion of the panel base part 70 and a motor (not shown) that drives the front wheels 73 and the rear wheels 74. The panel traveling mechanism 72 may be adapted to travel by being pushed by, for example, a user, instead of being driven by the motor. The front wheels 73 and the rear wheels 74 are an example of a plurality of wheels of the present disclosure.

The panel support part 64 extends in a direction (Z direction) intersecting the floor surface F and comprises an arm 66. The panel support part 64 is a part that forms a body part of the panel unit 60, and a controller for controlling the entire panel unit 60, a mechanism for driving the arm 66, and the like are accommodated in the panel support part 64. Further, the panel support part 64 is connected to the arm 66 at a connecting portion 67 adapted such that the position of the connecting portion 67 in the height direction can be changed.

The arm 66 is a part extending from the panel support part 64, one end of the arm 66 is mounted on the panel support part 64, and the panel 62 is provided at the other end of the arm 66. The arm 66 is extendable and contractible, and is rotatable about an axis extending in the right-left direction (X direction) at the connecting portion 67 between the arm 66 and the panel support part 64. That is, the panel 62 is adapted such that the position of the panel 62 in the height direction (Z direction) perpendicular to the floor surface F can be changed due to the extension and contraction of the arm 66 and the rotation of the arm 66 about the axis extending in the right-left direction.

Further, the arm 66 is adapted to be capable of changing an orientation of the panel 62 at a connecting portion 68 between the arm 66 and the panel 62. For example, the connecting portion 68 may be to support the panel 62 in an orientation in which a radiation detection surface of the panel 62 is perpendicular to the floor surface F regardless of a state where the arm 66 extends or contracts or is rotated, or may be capable of fixing the panel 62 in any orientation.

A form of the arm 66 is not particularly limited. For example, the arm 66 may be an L-shaped arm as viewed from the right side (that is, the same form as the arm 46 of the radiation source unit 40 shown in Fig. 1), may be a C-shaped arm as viewed from the right side, or may be an arm including a plurality of joints.

The radiography system 10 according to the present embodiment is not limited to being applied to imaging in a standing position, and can also be applied to imaging in a supine position, a sitting position, and the like. Another use aspect of the radiography system 10 shown in Fig. 1 is shown in Fig. 2 as an example. An aspect in which the subject A in a supine position is imaged is shown in an example shown in Fig. 2. In a case where the subject A in the supine position is imaged, the panel 62 of the panel unit 60 is detached and then attached to the inside of a supine table 77 and the subject A placed on the supine table 77 is irradiated with the radiation R from above, so that imaging is performed. That is, the panel 62 of the panel unit 60 may be attachable to and detachable from the arm 66.

In a case where such a portable radiography system 10 is operated, it is considered to store each unit in an appropriate place when the unit is not used and to carry out each unit only when the unit is used. Therefore, in the radiography system 10 according to the present embodiment, the radiation source unit 40 and the panel unit 60 are adapted such that storage efficiency thereof is improved.

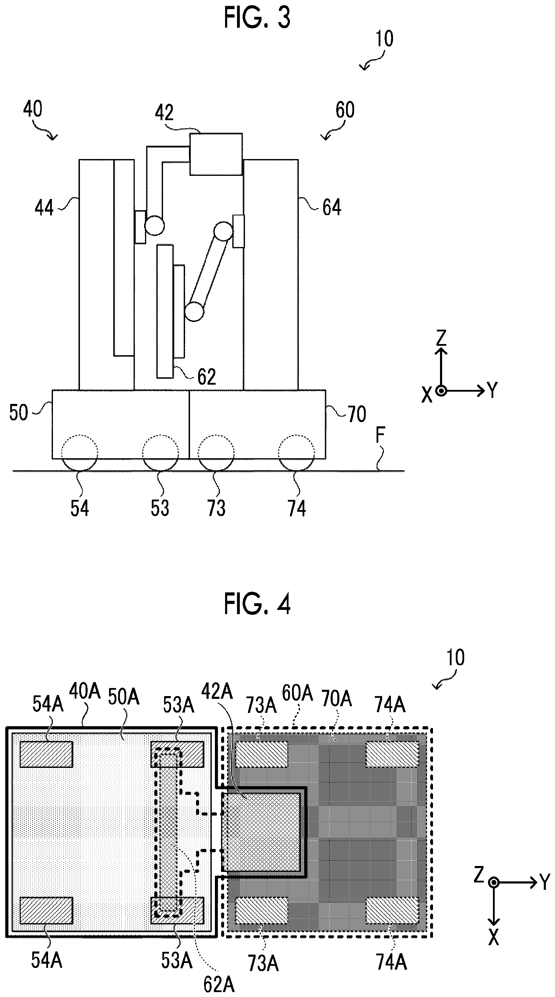

Detailed configurations of the radiation source unit 40 and the panel unit 60 will be described below with reference to Figs. 3 and 4. Fig. 3 is a schematic diagram showing an example of a state of the radiation source unit 40 and the panel unit 60 that can be reproduced in a case where the radiography system 10 is stored. Fig. 4 is a diagram showing respective projection regions in which the respective components of the radiation source unit 40 and the panel unit 60 in the state shown in Fig. 3 are projected in a direction perpendicular to the floor surface F in a state where the floor surface F is defined as a projection surface.

In Fig. 4, the projection region of the radiation source unit 40 is shown as the entire radiation source region 40A. The projection region of the radiation source base part 50 is shown as a radiation source base region 50A. The projection region of the radiation emitting unit 42 is shown as an emitting unit region 42A. The respective projection regions of the front wheels 53 and the rear wheels 54 of the radiation source unit 40 are shown as wheel regions 53A and 54A. The projection region of the panel unit 60 is shown as the entire panel region 60A. The projection region of the panel base part 70 is shown as a panel base region 70A. The projection region of the panel 62 is shown as a panel region 62A. The respective projection regions of the front wheels 73 and the rear wheels 74 of the panel unit 60 are shown as wheel regions 73A and 74A. An outer edge of the projection region related to the radiation source unit 40 is shown by a solid line, and an outer edge of the projection region related to the panel unit 60 is shown by a dotted line. In Fig. 4, for easy understanding, portions in which the outer edges of two projection regions overlap with each other (for example, the left side of the entire radiation source region 40A on the plane of paper and the left side of the radiation source base region 50A on the plane of paper) are shown with an offset.

The radiation source base region 50A and the panel base region 70A have a common shape. That is, the shapes of occupied spaces of the radiation source base part 50 and the panel base part 70 on the floor surface F are the same as each other. The radiation source base region 50A and the panel base region 70A only have to have a common shape (for example, a rectangular shape) as a whole, and may have an error that is generally allowed in the technical field to which the technique of the present disclosure belongs. For example, an error in the dimensional ratio, an error in the radius of a round portion of a chamfered portion of a corner, and the like are allowed.

Further, the radiography system 10 is adapted such that the radiation source unit 40 and the panel unit 60 can be disposed in a close state where at least one of the entire radiation source region 40A and the panel base region 70A or the entire panel region 60A and the radiation source base region 50A at least partially overlap with each other. Specifically, the radiography system 10 is adapted to be capable of taking the close state in a case where the radiation source unit 40 is in a contracting state and the panel unit 60 is in a contracting state. The contracting state of the radiation source unit 40 is a state where a length, an orientation, and the like of the arm 46 or the like are adjusted such that the area of the entire radiation source region 40A is the smallest. The contracting state of the panel unit 60 is a state where a length, an orientation, and the like of the arm 66 or the like are adjusted such that the area of the entire panel region 60A is the smallest.

Specifically, the radiography system 10 is adapted such that the radiation emitting unit 42 and the panel 62 can be made not to interfere with each other in the height direction in the close state due to the functions of the radiation source support part 44, the panel support part 64, and the like. Moreover, the radiography system 10 is adapted to be capable of taking a state where any one or both of a state where a part of the radiation source unit 40 is positioned above the panel base region 70A and a state where a part of the panel unit 60 is positioned above the radiation source base part 50 are satisfied.

In the example shown in Fig. 3, the radiography system 10 takes a state where a part of the radiation source unit 40, particularly, the radiation emitting unit 42 is positioned above the panel base region 70A and a state where a part of the panel unit 60, particularly, the panel 62 is positioned above the radiation source base part 50. In correspondence with this state, in an example shown in Fig. 4, the entire radiation source region 40A and the panel base region 70A, specifically, the emitting unit region 42A and the panel base region 70A at least partially overlap with each other in the close state. Further, the entire panel region 60A and the radiation source base region 50A, specifically, the panel region 62A and the radiation source base region 50A at least partially overlap with each other in the close state.

According to such a configuration, it is possible to reduce an occupied space of one set of the radiation source unit 40 and the panel unit 60 and to reduce a dead space. Therefore, it is possible to improve the storage efficiency of the radiography system 10.

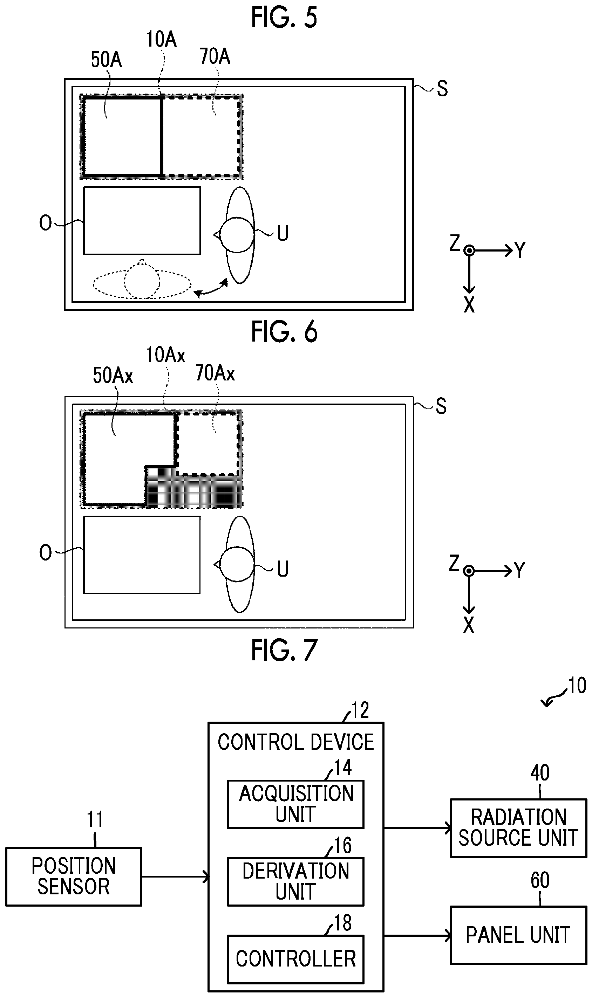

An example of a state where the radiography system 10 according to the present embodiment is stored in a storage place S is shown in Fig. 5. Fig. 5 is a plan view of the storage place S (for example, a room in a medical institution) as viewed from above, and shows the radiation source base region 50A, the panel base region 70A, and an occupied space 10A of the entire radiography system 10 (that is, one set of the radiation source unit 40 and the panel unit 60). Further, an example of a state where a radiography system according to a comparative example is stored in the same storage place S as in Fig. 5 is shown in Fig. 6. The shapes of an occupied space of a radiation source unit 40 (radiation source base region 50Ax) and an occupied space of a panel unit 60 (panel base region 70Ax) in the radiography system according to the comparative example are different from those in the present embodiment.

According to the radiography system 10 of the present embodiment, the total occupied space 10A of the radiation source unit 40 and the panel unit 60 can be made smaller than an occupied space 10Ax of the comparative example. Further, a dead space present in the occupied space 10Ax can be reduced. Therefore, storage efficiency in the storage place S can be improved, so that a large number of devices (for example, another set of a radiation source unit 40 and a panel unit 60, and another device O, and the like) can be stored. Furthermore, since, for example, a space in which a user U can freely move can be expanded, it is advantageous in securing a workspace for the operation, transport, and the like of each device. As a result, it is possible to effectively use the storage place S.

The occupied space can also be reduced through a reduction in the size of the radiation source unit 40 and/or the panel unit 60. However, since each unit is required to have a footprint large enough to prevent the unit from being overturned, a reduction in size is limited. According to the radiography system 10 of the present embodiment, the radiation source base region 50A and the panel base region 70Ahave a common shape, so that a dead space is reduced. Therefore, even in a case where a size large enough to prevent the unit from being overturned is maintained, storage efficiency can be improved.

Further, according to the radiography system 10 of the present embodiment, it is possible to store the radiation source unit 40 and the panel unit 60 in an appropriate storage mode (close state) having a small occupied space and a small dead space without largely transforming the radiation source unit 40 and the panel unit 60. Therefore, the switching between a storage state and a use state can be quickly performed. Furthermore, each unit is easily taken in and out of the storage place S.

It is preferable that the radiation source base region 50A and the panel base region 70A have a congruent shape. That is, it is preferable that the radiation source base part 50 and the panel base part 70 have occupied spaces having the same size and shape on the floor surface F. According to such a configuration, a dead space can be further reduced, so that it is advantageous in improving storage efficiency. The sizes and shapes of the radiation source base region 50A and the panel base region 70A only have to be the same as each other as a whole, and may have an error that is generally allowed in the technical field to which the technique of the present disclosure belongs. For example, an error in the dimensional ratio, an error in the radius of a round portion of a chamfered portion of a corner, and the like are allowed.

Further, it is preferable that the radiation source base region 50A and the panel base region 70A have a rectangular shape. According to such a configuration, it is advantageous in reducing a dead space, so that it is advantageous in improving storage efficiency. The rectangular shape referred to here does not need to be a perfect rectangular shape, and may be a substantially rectangular shape, for example, a rectangular shape having a chamfered portion at a corner.

Furthermore, it is preferable that the total region of the entire radiation source region 40A and the entire panel region 60A can have a rectangular shape in the close state. Specifically, it is preferable that three sides (an upper side, a left side, and a lower side in the plane of paper of Fig. 4) of the entire radiation source region 40A other than a side (a right side in the plane of paper of Fig. 4) thereof protruding toward the entire panel region 60A in the close state do not exceed the range of the radiation source base region 50A. Similarly, it is preferable that three sides (an upper side, a right side, and a lower side in the plane of paper of Fig. 4) of the entire panel region 60A other than a side (a left side in the plane of paper of Fig. 4) thereof protruding toward the entire radiation source region 40A in the close state do not exceed the range of the panel base region 70A. According to such a configuration, a dead space can be further reduced, so that it is advantageous in improving storage efficiency. The rectangular shape referred to here does not need to be a perfect rectangular shape, and may be a substantially rectangular shape, for example, a rectangular shape having a chamfered portion at a corner. In addition, for example, a gap portion may be provided between the radiation source base region 50A and the panel base region 70A, or a notched portion may be provided between the entire radiation source region 40A and the entire panel region 60A.

Further, it is preferable that each of the radiation source base part 50 and the panel base part 70 includes a plurality of wheels disposed in a common arrangement pattern. In the example shown in Fig. 4, the radiation source base part 50 includes four wheels (the front wheels 53 and the rear wheels 54) disposed at four corners of the radiation source base region 50A. Similarly, the panel base part 70 includes four wheels (the front wheels 73 and the rear wheels 74) disposed at four corners of the panel base region 70A. Since the radiation source unit 40 and the panel unit 60 have a common structure as described above, the productivity of the radiography system 10 can be improved.

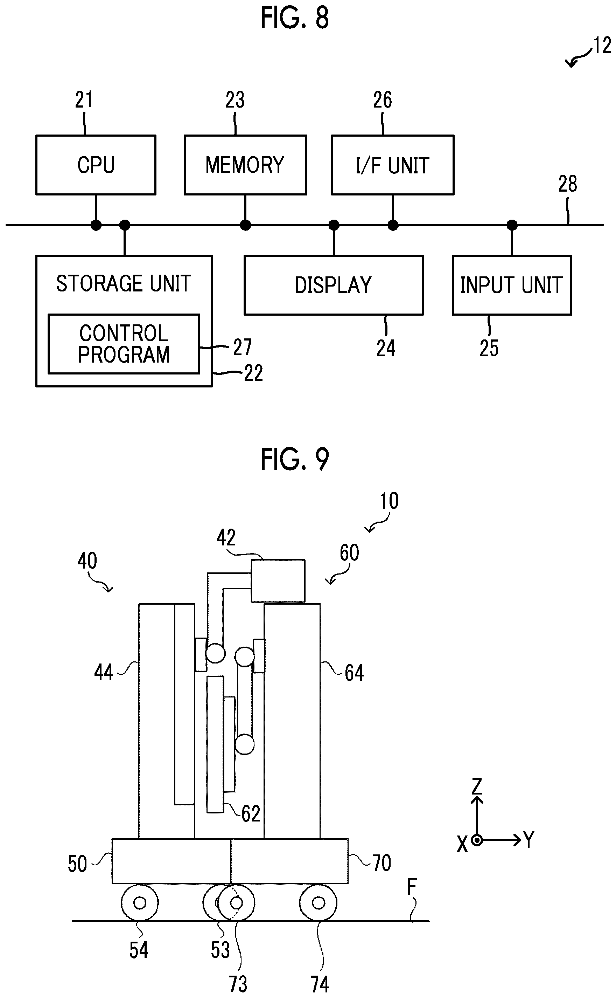

Next, positioning processing in the height direction in the radiography system 10 according to the present embodiment will be described with reference to Figs. 7 and 8. The radiography system 10 according to the present embodiment may have a function of aligning the position of the radiation emitting unit 42 with the position of the panel 62 in the height direction in a separated state (see Fig. 1) where the radiation source unit 40 and the panel unit 60 are disposed to be separated from each other for radiography. The separated state is a state where the respective regions of the entire radiation source region 40A and the panel base region 70A do not overlap with each other and the respective regions of the entire panel region 60A and the radiation source base region 50A do not overlap with each other.

Fig. 7 is an example of a functional block diagram showing a functional configuration of the radiography system 10 according to the present embodiment, particularly, a control device 12. The radiography system 10 may comprise a position sensor 11 that detects a position of each unit in the height direction and a control device 12 that controls the position of each unit in the height direction, in addition to the radiation source unit 40 and the panel unit 60.

The position sensor 11 is a sensor that is used to obtain difference information indicating a difference between the positions of the radiation emitting unit 42 and the panel 62 in the height direction in the separated state. For example, a distance-measuring sensor that measures a distance between the sensor and an object, a displacement sensor that measures a displacement from a predetermined reference position, and the like can be appropriately applied as the position sensor 11. For example, a laser imaging detection and ranging or light detection and ranging (LIDAR), a time-of-flight (TOF) camera, a stereo camera, and the like can be applied as the distance-measuring sensor. Examples of the displacement sensor include a linear potentiometer, a laser displacement meter, and the like.

For example, a distance-measuring sensor disposed at a predetermined position in a radiography room may be used as the position sensor 11 to detect both the position of the radiation emitting unit 42 and the position of the panel 62 and to obtain a difference therebetween. Further, for example, a distance-measuring sensor mounted on any one of the radiation source unit 40 or the panel unit 60 may be used as the position sensor 11 to detect a difference between the position of the radiation emitting unit 42 or the panel 62 and the position of a radiation emitting unit 42 or a panel 62 of the other unit. Furthermore, for example, displacement sensors provided on the radiation source support part 44 and the panel support part 64 may be used as the position sensor 11 to detect both the displacement of the radiation emitting unit 42 and the displacement of the panel 62 and to obtain a difference therebetween may be obtained.

The control device 12 controls the radiation source unit 40 and/or the panel unit 60 on the basis of the difference information obtained by the position sensor 11.

First, an electrical configuration of the control device 12 will be described with reference to Fig. 8. The control device 12 includes a central processing unit (CPU) 21, a nonvolatile storage unit 22, and a memory 23 as a temporary storage area. Further, the control device 12 includes a display 24, such as a liquid crystal display, an input unit 25, such as a keyboard and a mouse, and an interface (I/F) unit 26. The I/F unit 26 performs wired and/or wireless communication with the position sensor 11, the radiation source unit 40, the panel unit 60, and other external devices. The CPU 21, the storage unit 22, the memory 23, the display 24, the input unit 25, and the I/F unit 26 are connected to be capable of exchanging various types of information with each other through a bus 28 such as a system bus and a control bus.

The storage unit 22 is realized by, for example, a storage medium, such as a hard disk drive (HDD), a solid state drive (SSD), and a flash memory. A control program 27 for the control device 12 is stored in the storage unit 22. The CPU 21 reads out the control program 27 from the storage unit 22, develops the control program 27 into the memory 23, and executes the developed control program 27. The CPU 21 is an example of a processor of the present disclosure. For example, a personal computer, a server computer, a smartphone, a tablet terminal, a wearable terminal, and the like can be appropriately applied as the control device 12.

Next, a functional configuration of the control device 12 will be described with reference to Fig. 7. The control device 12 includes an acquisition unit 14, a derivation unit 16, and a controller 18. In a case where the CPU 21 executes the control program 27, the CPU 21 functions as the acquisition unit 14, the derivation unit 16, and the controller 18.

The acquisition unit 14 acquires difference information indicating a difference between the positions of the radiation emitting unit 42 and the panel 62 in the height direction in the separated state. For example, the acquisition unit 14 may acquire information indicating the position of the radiation emitting unit 42 in the height direction and information indicating the position of the panel 62 in the height direction from the position sensor 11, and may derive a difference therebetween.

The derivation unit 16 derives a moving distance of at least one of the radiation emitting unit 42 or the panel 62 in the height direction on the basis of the difference information acquired by the acquisition unit 14 such that a positional relationship between the radiation emitting unit 42 and the panel 62 in the height direction is a predetermined state. Specifically, the derivation unit 16 derives the moving distance of the radiation emitting unit 42 and/or the panel 62 in the height direction with a goal to make a state where a center of the radiation R emitted from the radiation emitting unit 42 and a center of the panel 62 coincide with each other.

For example, it is assumed that the position of the panel 62 is determined on the basis of a part to be imaged, a physique, a posture, and the like of the subject A. In this case, the derivation unit 16 derives the moving distance of the radiation emitting unit 42 in the height direction such that an optical axis of the radiation R is aligned with the center of the radiation detection surface of the panel 62.

The controller 18 controls the radiation source unit 40 and/or the panel unit 60 on the basis of the moving distance derived by the derivation unit 16 to change the position of the radiation emitting unit 42 and/or the panel 62 in the height direction.

The present disclosure is not limited to an aspect in which the control device 12 controls the radiation source unit 40 and/or the panel unit 60, and may include, for example, an aspect in which a user manually changes the position of the radiation emitting unit 42 and/or the panel 62 in the height direction. Specifically, the controller 18 may cause the display 24 to display the moving distance derived by the derivation unit 16, and a user who sees the displayed moving distance may manually adjust the position of the radiation emitting unit 42 and/or the panel 62 in the height direction.

As described above, the radiography system 10 according to the present embodiment comprises the radiation source unit 40 and the panel unit 60. The radiation source unit 40 comprises the radiation emitting unit 42 that includes the radiation source 43, and the radiation source base part 50 that supports the radiation emitting unit 42 and can travel on the floor surface F. The panel unit 60 comprises the panel 62 that detects the radiation R emitted from the radiation emitting unit 42, and the panel base part 70 that supports the panel 62 and can travel on the floor surface F. The floor surface F is defined as a projection surface, the projection region of the radiation source unit 40 is defined as the entire radiation source region 40A, the projection region of the radiation source base part 50 is defined as the radiation source base region 50A, the projection region of the panel unit 60 is defined as the entire panel region 60A, and the projection region of the panel base part 70 is defined as the panel base region 70A. In this case, the radiation source base region 50A and the panel base region 70A have a common shape. Further, the radiation source unit 40 and the panel unit 60 can be disposed in the close state where at least one of the entire radiation source region 40A and the panel base region 70A or the entire panel region 60A and the radiation source base region 50A at least partially overlap with each other.

That is, according to the radiography system 10 of the present embodiment, the radiation source unit 40 and the panel unit 60 can be disposed in the close state where the entire region of one of the radiation source unit 40 and the panel unit 60 overlaps with the base region of the other thereof. That is, since the radiography system 10 can be adapted such that a part of the radiation source unit 40 and a part of the panel unit 60 are accommodated in a common region, the occupied space of the entire radiography system 10 can be reduced. Further, since the radiation source base region 50A and the panel base region 70A have a common shape, it is possible to reduce a dead space in the close state. Therefore, according to the radiography system 10 of the present embodiment, storage efficiency can be improved.

An aspect in which the positions of both the radiation emitting unit 42 and the panel 62 in the height direction can be changed has been described in the above-described embodiment, but the present disclosure is not limited thereto. In order to avoid interference between the radiation emitting unit 42 and the panel 62 in the close state, at least one of the radiation emitting unit 42 or the panel 62 only has to be adapted such that the position thereof in the height direction can be changed.

Further, an aspect in which each of both the radiation emitting unit 42 and the panel 62 is positioned above the base part of the other thereof has been described in the above-described embodiment. However, the present disclosure is not limited thereto, and any one of the radiation emitting unit 42 or the panel 62 may be positioned above the base part of the other thereof. For example, while the radiation emitting unit 42 is positioned above the panel base part 70, the panel 62 may also be positioned above the panel base part 70 (that is, the panel 62 may not be positioned above the radiation source base part 50). Similarly, for example, while the panel 62 is positioned above the radiation source base part 50, the radiation emitting unit 42 may also be positioned above the radiation source base part 50 (that is, the radiation emitting unit 42 may not be positioned above the panel base part 70).

Furthermore, an aspect in which the control device 12 is a device connected to the radiation source unit 40 and the panel unit 60 by wire and/or wirelessly has been described in the above-described embodiment, but the present disclosure is not limited thereto. For example, the control device 12 may be mounted on the radiation source unit 40 and/or the panel unit 60.

### First modification example

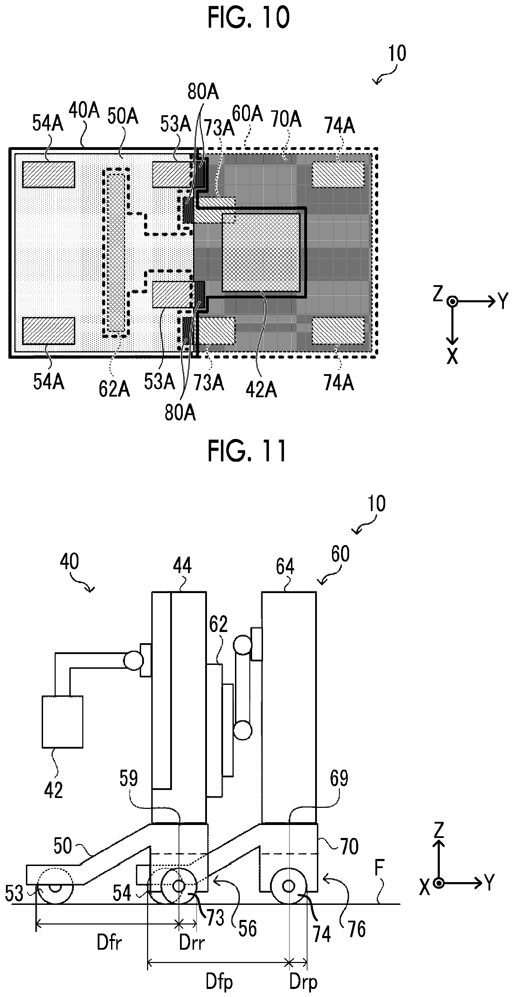

A first modification example of the radiography system 10 will be described with reference to Figs. 9 and 10. A radiography system 10 according to the first modification example is adapted such that the radiation source base region 50A and the panel base region 70A include overlap regions 80A in which the radiation source base region 50A and the panel base region 70A at least partially overlap with each other in the close state. That is, in addition to the radiation emitting unit 42 and the panel 62, a part of each base part is also adapted to be accommodated in a region common to the base part of the counter unit. The description of the same configuration as that of the above-described embodiment will be partially omitted below.

Fig. 9 is a schematic diagram showing an example of a state of the radiation source unit 40 and the panel unit 60 that can be reproduced in a case where the radiography system 10 according to the first modification example is stored. Fig. 10 is a diagram showing each projection region in which each of components of the radiation source unit 40 and the panel unit 60 in the state shown in Fig. 9 is projected in a state where the floor surface F is defined as a projection surface.

The radiography system 10 according to the first modification example is adapted such that each of a part of the wheels of the radiation source unit 40 and a part of the wheels of the panel unit 60 can enter under a base part of the counter unit. Specifically, the mounting positions of the wheels of the radiation source base part 50 and the wheels of the panel base part 70 are made different from each other in a width direction, so that the above configuration is realized. The width direction is a direction (X direction) intersecting a direction (Y direction) in which the radiation source unit 40 and the panel unit 60 face each other on the floor surface F in the close state.

One front wheel 53 of the radiation source unit 40 is disposed to be shifted toward a middle in the width direction in an example shown in Fig. 10, so that the front wheel 53 does not interfere with the front wheel 73 of the panel unit 60 in the close state. Similarly, one front wheel 73 of the panel unit 60 is disposed to be shifted toward a middle in the width direction, so that the front wheel 73 does not interfere with the front wheel 53 of the radiation source unit 40 in the close state.

In this case, as shown in Fig. 10, in the close state, a part (wheel regions 73A) of the wheel regions of the panel base part 70 enter the radiation source base region 50A, and a part (wheel regions 53A) of the wheel regions of the radiation source base part 50 enter the panel base region 70A. That is, some wheel regions and the overlap regions 80A at least partially overlap with each other in the close state.

According to the radiography system 10 of the present modification example, in the close state, a part of the wheels of the radiation source unit 40 and/or a part of the wheels of the panel unit 60 can enter under the base part of the counter unit. Accordingly, an occupied space can be further reduced. Therefore, according to the radiography system 10 of the present modification example, storage efficiency can be improved.

An aspect in which the wheels of each unit enter under the base part of the counter unit has been described in the first modification example, but the present disclosure is not limited thereto. Only the wheels of any one unit may enter under the base part of the other unit. For example, only the front wheels 53 of the radiation source unit 40 may be adapted to be capable of entering under the panel base part 70, and the front wheels 73 of the panel unit 60 may be adapted not to enter under the radiation source base part 50.

Further, Figs. 9 and 10 illustrate a state where the emitting unit region 42A and the panel base region 70A overlap with each other (a state where the radiation emitting unit 42 is positioned above the panel base region 70A) in the close state, but the present disclosure is not limited thereto. Similarly, Figs. 9 and 10 illustrate a state where the panel region 62A and the radiation source base region 50A overlap with each other (a state where the panel 62 is positioned above the radiation source base part 50) in the close state, but the present disclosure is not limited thereto. For example, the emitting unit region 42A and the panel base region 70A may not overlap with each other, and the panel region 62A and the radiation source base region 50A may not overlap with each other.

### Second modification example

A second modification example of the radiography system 10 will be described with reference to Figs. 11 to 13. A radiography system 10 according to the second modification example is adapted such that the radiation source base region 50A and the panel base region 70A include an overlap region 80A in which the radiation source base region 50A and the panel base region 70A at least partially overlap with each other in the close state. Second modification example is different from the first modification example in that the base parts other than the wheels are also adapted to be accommodated in a common region. The description of the same configuration as that of the above-described embodiment will be partially omitted below.

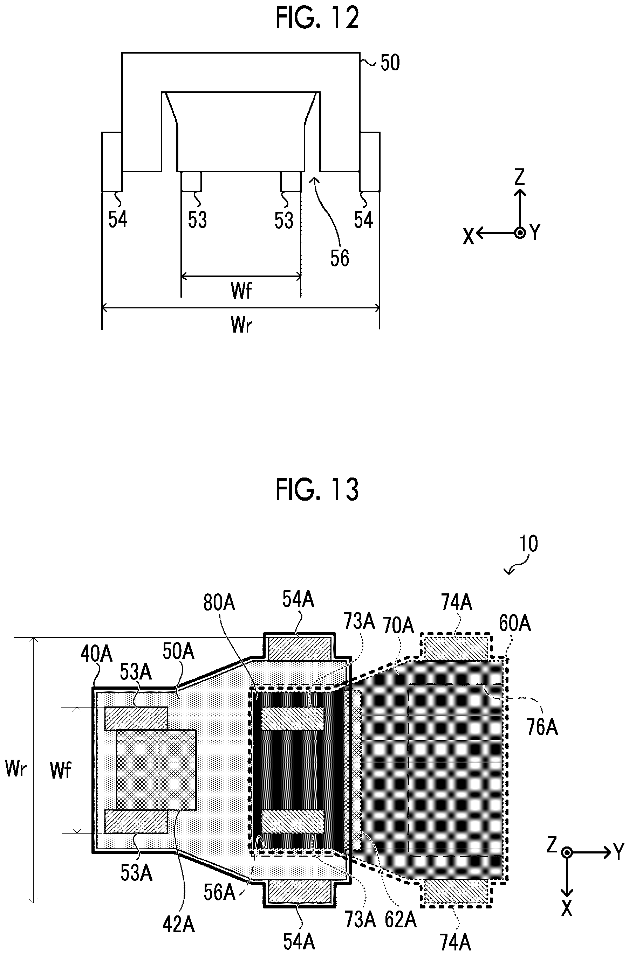

Fig. 11 is a schematic diagram showing an example of a state of the radiation source unit 40 and the panel unit 60 that can be reproduced in a case where the radiography system 10 according to the second modification example is stored. Fig. 12 is a diagram of the radiation source base part 50 as viewed from a rear wheel 54 side. Fig. 13 is a diagram showing each projection region in which each of components of the radiation source unit 40 and the panel unit 60 in the state shown in Fig. 11 is projected in a state where the floor surface F is defined as a projection surface.

In the close state (storage state) in the above-described embodiment and the first modification example, the orientations of the radiation source unit 40 and the panel unit 60 were the same as the orientations thereof in the separated state (use state) as shown in Fig. 1. That is, the radiation emitting unit 42 and the panel 62 were disposed to face each other by facing in opposite directions. On the other hand, the orientation of the radiation source unit 40 is reversed in the close state in the second modification example. That is, the radiation emitting unit 42 and the panel 62 are disposed to face the same direction (left in the plane of paper of Fig. 11).

The radiation source base part 50 includes one or more front wheels 53 and two or more rear wheels 54. Similarly, the panel base part 70 includes one or more front wheels 73 and two or more rear wheels 74. In the examples shown in Figs. 11 to 13, the radiation source base part 50 includes two front wheels 53 and two rear wheels 54. The panel base part 70 includes two front wheels 73 and two rear wheels 74. Here, a width Wf of all the front wheels 53 (front wheels 73) in the width direction (X direction) is smaller than a width Wr of all the rear wheels 54 (rear wheels 74) in the width direction. That is, here, the wheels having a smaller total width are referred to as front wheels.

At least a part of the front wheels 73 included in the panel traveling mechanism 72 are adapted to enter between the rear wheels 54 included in the radiation source base part 50 in the close state. Specifically, the height of the radiation source base part 50 is increased from a front wheel 53 side to the rear wheel 54 side, and an opening portion 56 is provided between the rear wheels 54. The configuration of the panel base part 70 is also the same as the configuration of the radiation source base part 50. The height of the panel base part 70 is increased from a front wheel 73 side to a rear wheel 74 side, and an opening portion 76 is provided between the rear wheels 74.

That is, the front wheels 53 and 73 are disposed on an inner side such that the total widths of the front wheels 53 and 73 are smaller than the total widths of the rear wheels 54 and 74, respectively, and the base parts around the front wheels 53 and 73 are relatively low. With these configurations, the front wheel 73 side of the panel base part 70 can enter the opening portion 56 of the radiation source base part 50 without interference between the base parts.

In this case, as shown in Fig. 13, some wheel regions and the overlap region 80A at least partially overlap with each other in the close state. Positions corresponding to the heights of the opening portion 56 and the opening portion 76 are shown in Fig. 11 by a broken line. Further, a range 56A and a range 76A where the other unit can enter through the opening portion 56 and the opening portion 76 are shown in Fig. 13 by a broken line.

An aspect in which a part of the panel base part 70 enters under the radiation source base part 50 is illustrated in Figs. 11 to 13, but the present disclosure is not limited thereto. For example, the positions of the radiation source unit 40 and the panel unit 60 may be exchanged such that a part of the radiation source base part 50 enters under the panel base part 70.

Specifically, at least a part of the front wheels 53 included in the radiation source base part 50 may be adapted to enter between the rear wheels 74 included in the panel base part 70 in the close state. For example, the front wheel 53 side of the radiation source base part 50 may be allowed to enter toward the opening portion 76 provided on the rear wheel 74 side of the panel base part 70.

Further, the opening portion 56 and the opening portion 76 may be provided in both the radiation source base part 50 and the panel base part 70, or an opening portion may be provided only in the receiving-side base part (the radiation source base part 50 in Fig. 11). In a case where the opening portions are provided in both the base parts, at least a part of the front wheels 53 or the front wheels 73 included in any one of the radiation source base part 50 or the panel base part 70 can enter between the rear wheels 54 or the rear wheels 74 included in the other of the radiation source base part 50 and the panel base part 70 in the close state. That is, for example, in a case where the radiation source unit 40 and the panel unit 60 are to be stored, the close state (state suitable for storage) can be set regardless of the arrangement order. Therefore, convenience can be improved. Furthermore, for example, since the radiation source unit 40 and the panel unit 60 can be disposed in a chain in combination with a radiation source unit 40 and a panel unit 60 of another radiography system 10 while the base parts overlap with each other, it is advantageous in improving the storage efficiency.

In addition, it is preferable that the position of each support part is close to the rear wheel side in the radiography system 10 according to the second modification example. Specifically, it is preferable that, as shown in Fig. 11, a distance Drr from a connecting portion 59 between the radiation source base part 50 and the radiation source support part 44 to an end portion of the radiation source base part 50 corresponding to the rear wheel 54 is shorter than a distance Dfr from the connecting portion 59 to an end portion of the radiation source base part 50 corresponding to the front wheel 53 in a direction (Y direction) in which the radiation source unit 40 and the panel unit 60 face each other in the close state. Similarly, it is preferable that a distance Drp from a connecting portion 69 between the panel base part 70 and the panel support part 64 to an end portion of the panel base part 70 corresponding to the rear wheel 74 is shorter than a distance Dfp from the connecting portion 69 to an end portion of the panel base part 70 corresponding to the front wheel 73 in a direction (Y direction) in which the radiation source unit 40 and the panel unit 60 face each other in the close state.

Since each support part is close to the high rear wheel side of the base part, it is possible to avoid interference between the radiation source unit 40 and the panel unit 60 in the close state. Further, since each of the radiation emitting unit 42 and the panel 62 can be lifted to a higher position by the height of the base part, it is an advantageous in radiography. Furthermore, since the support part is not provided on the front wheel side, the front wheel side of the base part can be placed at a deeper position under the supine table 77 in a case where radiography is performed in a supine position (see Fig. 2). Therefore, convenience is improved.

According to the radiography system 10 of the present modification example, a part of the panel base part 70 and/or a part of the radiation source base part 50 can enter under the counter unit in the close state. Accordingly, an occupied space can be further reduced. Therefore, according to the radiography system 10 of the present modification example, storage efficiency can be improved.

In the second modification example, the opening portions may not be provided in both the radiation source base part 50 and the panel base part 70. For example, only the radiation source base part 50 may include the opening portion 56, and the panel base part 70 may not include the opening portion 76 (that is, may not allow the other unit to enter).

Further, whether or not the emitting unit region 42A and the panel base region 70A overlap with each other in the close state and whether or not the panel region 62A and the radiation source base region 50A overlap with each other in the close state are not particularly limited in the above-described second modification example.

The forms of the wheels shown in the above-described embodiment and each modification example are merely examples, and can be modified in various ways. For example, the number of front wheels and/or the number of rear wheels may be independently changed. For example, one front wheel and two rear wheels may be combined or two front wheels and three rear wheels may be combined. Further, for example, the sizes and shapes of the front wheels and the rear wheels may be changed.

In addition, in the above-described embodiment, for example, various processors to be described below can be used as the hardware structures of processing units, which perform various types of processing, such as the acquisition unit 14, the derivation unit 16, and the controller 18. The various processors described above include a programmable logic device (PLD) that is a processor of which the circuit configuration can be changed after manufacture, such as a field programmable gate array (FPGA), a dedicated electrical circuit that is a processor having a circuit configuration designed exclusively to perform specific processing, such as an Application Specific Integrated Circuit (ASIC), and the like in addition to a CPU that is a general-purpose processor functioning as various processing units by executing software (program) as described above.

One processing unit may be formed of one of these various processors, or may be formed of a combination of two or more processors of the same type or different types (for example, a combination of a plurality of FPGAs or a combination of a CPU and an FPGA). Further, a plurality of processing units may be formed of one processor.

As an example where a plurality of processing units are formed of one processor, first, there is an aspect in which one processor is formed of a combination of one or more CPUs and software as typified by a computer, such as a client or a server, and functions as a plurality of processing units. Second, there is an aspect in which a processor realizing the functions of the entire system, which includes a plurality of processing units, by one integrated circuit (IC) chip as typified by System On Chip (SoC) or the like is used. In this way, various processing units are formed using one or more of the above-described various processors as hardware structures.

In addition, more specifically, electrical circuitry where circuit elements, such as semiconductor elements, are combined can be used as the hardware structures of these various processors.

Further, an aspect in which various programs are stored (installed) in advance in the storage unit has been described in the above-described embodiment, but the present disclosure is not limited thereto. The various programs may be recorded on a recording medium, such as a compact disc read only memory (CD-ROM), a digital versatile disc read only memory (DVD-ROM), or a universal serial bus (USB) memory, and then provided. The various programs may be provided in a form of being downloaded from an external device via a network. Furthermore, the technique of the present disclosure extends to a storage medium that non-transitorily stores a program in addition to the program.

The technique of the present disclosure can also be appropriately combined with the embodiment and Examples. The description contents and shown contents having been described above are the detailed description of portions according to the technique of the disclosure, and are merely an example of the technique of the disclosure. For example, the description of the configuration, functions, actions, and effects having been described above is the description of examples of the configuration, functions, actions, and effects of the portions according to the technique of the disclosure. Accordingly, it goes without saying that unnecessary portions may be deleted or new elements may be added or replaced in the description contents and shown contents described above without departing from the scope of the invention as defined by the claims.

## Claims

1. A radiography system comprising:
a radiation source unit (40); and
a panel unit (60), wherein:
the radiation source unit includes a radiation emitting unit (42) that includes a radiation source (43), and a radiation source base part (50) that supports the radiation emitting unit and is capable of traveling on a floor surface,
the panel unit includes a panel (62) that detects radiation emitted from the radiation emitting unit, and a panel base part (70) that supports the panel and is capable of traveling on the floor surface,
in a case where the floor surface is defined as a projection surface:
a projection region of the radiation source unit is defined as an entire radiation source region;
a projection region of the radiation source base part is defined as a radiation source base region;
a projection region of the panel unit is defined as an entire panel region; and
a projection region of the panel base part is defined as a panel base region, the radiation source base region and the panel base region have a common shape, and
the radiation source unit and the panel unit are capable of being disposed in a close state where at least one of the entire radiation source region and the panel base region or the entire panel region and the radiation source base region at least partially overlap with each other.

2. The radiography system according to claim 1, wherein the entire radiation source region and the panel base region at least partially overlap with each other in the close state.

3. The radiography system according to claim 2, wherein, in a case where the floor surface is defined as the projection surface and a projection region of the radiation emitting unit is defined as an emitting unit region, the emitting unit region and the panel base region at least partially overlap with each other in the close state.

4. The radiography system according to any one of claims 1 to 3, wherein the entire panel region and the radiation source base region at least partially overlap with each other in the close state.

5. The radiography system according to claim 4, wherein, in a case where the floor surface is defined as the projection surface and a projection region of the panel is defined as a panel region, the panel region and the radiation source base region at least partially overlap with each other in the close state.

6. The radiography system according to any one of claims 1 to 5, wherein, in the close state:
the entire radiation source region and the panel base region at least partially overlap with each other, and
the entire panel region and the radiation source base region at least partially overlap with each other.

7. The radiography system according to claim 6, wherein, in a case where the floor surface is defined as the projection surface, a projection region of the radiation emitting unit is defined as an emitting unit region, and a projection region of the panel is defined as a panel region, in the close state:
the emitting unit region and the panel base region at least partially overlap with each other, and
the panel region and the radiation source base region at least partially overlap with each other.

8. The radiography system according to claim 6 or 7, wherein the radiation source base region and the panel base region include an overlap region in which the radiation source base region and the panel base region at least partially overlap with each other in the close state.

9. The radiography system according to claim 8, wherein:
each of the radiation source base part and the panel base part includes a plurality of wheels disposed in a common arrangement pattern, and
in a case where the floor surface is defined as the projection surface and a projection region of each of the plurality of wheels is defined as a wheel region, some of the wheel regions and the overlap region at least partially overlap with each other in the close state.

10. The radiography system according to claim 9, wherein, in a case where a direction intersecting a direction in which the radiation source unit and the panel unit face each other on the floor surface in the close state is defined as a width direction, mounting positions of the wheels of the radiation source base part and the wheels of the panel base part are different from each other in the width direction.

11. The radiography system according to claim 10, wherein, in the close state:
a part of the wheel regions of the panel base part enter the radiation source base region, and
a part of the wheel regions of the radiation source base part enter the panel base region.

12. The radiography system according to claim 9, wherein:
each of the radiation source base part and the panel base part includes one or more front wheels and two or more rear wheels, and
at least a part of the front wheels included in any one of the radiation source base part or the panel base part enter between the rear wheels included in the other of the radiation source base part and the panel base part in the close state.

13. The radiography system according to claim 12, wherein, in a case where a direction intersecting a direction in which the radiation source unit and the panel unit face each other on the floor surface in the close state is defined as a width direction, a width of all the front wheels in the width direction is smaller than a width of all the rear wheels in the width direction.

14. The radiography system according to claim 12 or 13, wherein:
the radiation source unit further includes a radiation source support part that extends in a direction intersecting the floor surface and connects the radiation source base part and the radiation emitting unit, and
a distance from a connecting portion between the radiation source base part and the radiation source support part to an end portion of the radiation source base part corresponding to the rear wheel is shorter than a distance from the connecting portion to an end portion of the radiation source base part corresponding to the front wheel in a direction in which the radiation source unit and the panel unit face each other in the close state.

15. The radiography system according to any one of claims 12 to 14, wherein:
the panel unit further includes a panel support part that extends in a direction intersecting the floor surface and connects the panel base part and the panel, and
a distance from a connecting portion between the panel base part and the panel support part to an end portion of the panel base part corresponding to the rear wheel is shorter than a distance from the connecting portion to an end portion of the panel base part corresponding to the front wheel in a direction in which the radiation source unit and the panel unit face each other in the close state.

16. The radiography system according to any one of claims 1 to 15, wherein the radiation source base region and the panel base region have a congruent shape.

17. The radiography system according to any one of claims 1 to 16, wherein the radiation source base region and the panel base region have a rectangular shape.

18. The radiography system according to claim 17, wherein a total region of the entire radiation source region and the entire panel region has a rectangular shape in the close state.

19. The radiography system according to any one of claims 1 to 18, wherein:
at least one of the radiation emitting unit or the panel is adapted such that a position of at least one of the radiation emitting unit or the panel in a height direction perpendicular to the floor surface is changeable, and
the radiation emitting unit and the panel do not interfere with each other in the height direction in the close state.

20. The radiography system according to claim 19, further comprising at least one processor, wherein the processor is configured to:
acquire difference information indicating a difference between positions of the radiation emitting unit and the panel in the height direction in a separated state where the respective regions do not overlap with each other; and
derive a moving distance of at least one of the radiation emitting unit or the panel in the height direction on the basis of the difference information such that a positional relationship between the radiation emitting unit and the panel in the height direction is a predetermined state.
